# EUROPÄISCHE PATENTSCHRIFT

(11) **EP 3 194 587 B1**
(45) Veröffentlichungstag und Bekanntmachung des Hinweises auf die Patenterteilung: **14.08.2019**
(21) Anmeldenummer: 15767083.7
(22) Anmeldetag: 14.08.2015
(51) Int. Cl.: C12N 9/88

(54) **LYASE UND FÜR DIE LYASE KODIERENDE DNA, DIE DNA ENTHALTENDE VEKTOREN, SOWIE VERFAHREN ZUR ASYMMETRISCHEN SYNTHESE VON (S)-PHENYLACETYLCARBINOL**
LYASES, DNA ENCODING THE LYASE, VECTOR BEARING THE DNA AND METHODS OF ASYMMETRICALLY PRODUCING (S)-PHENYLACETYLCARBINOL
LYASES, ADN CODANT LES LYASES, VECTEUR CONTENANT LEDIT ADN ET PROCÉDÉS POUR LA SYNTHÈSE ASYMÉTRIQUE DE (S)-PHENYLACETYLCARBINOL

(30) Priorität: 16.09.2014 DE 102014013644
(43) Veröffentlichungstag der Anmeldung: 26.07.2017
(73) Patentinhaber: Forschungszentrum Jülich GmbH, 52425 Jülich (DE)
(72) Erfinder: ROTHER, Dörte, 50354 Hürth (DE); POHL, Martina, 52066 Aachen (DE); SEHL, Torsten, 77654 Offenburg (DE); MARX, Lisa, 64342 Seeheim-Jugenheim (DE); WESTPHAL, Robert, 04155 Leipzig (DE)
(86) Internationale Anmeldenummer: PCT/DE2015/000407
(87) Internationale Veröffentlichungsnummer: WO 2016/041533

(56) Entgegenhaltungen:
- WO-A2-2010/039750
- US-A1- 2013 309 732
- ROTHER NEE GOCKE, DOERTE; KOLTER, GERALDINE; GERHARDS, TINA; BERTHOLD, CATRINE L; GAUCHENOVA, EKATERINA; KNOLL, MICHAEL; PLEISS, J: CHEMCATCHEM, Bd. 3, Nr. 10, 2011, Seiten 1587-1596, XP002751873, in der Anmeldung erwähnt

## Beschreibung

Die Erfindung betrifft eine Lyase und eine für die Lyase kodierende DNA, die DNA enthaltende Vektoren sowie ein Verfahren zur asymmetrischen Synthese von (*S*)-Phenylacetyl-carbinol.

(*S*)-Phenylacetylcarbinol ist ein wertvoller chiraler Baustein in organischen Synthesen und kann zur Synthese von Feinchemikalien und Pharmazeutika genutzt werden. Nach dem bisherigen Stand der Technik sind keine Methoden bekannt, bei den (*S*)-Phenylacetyl-carbinol (*S*)-PAC mittels asymmetrischer Synthese aus unchiralen, kostengünstigen Verbindungen in optischen Reinheiten >89 % ee generiert werden kann. Hohe optische Reinheiten sind jedoch von entschiedener Bedeutung bei der Herstellung von Feinchemikalien, bzw. Pharmazeutika.

Nach dem Stand der Technik sind verschiedene Methoden bekannt um (*S*)-Phenylacetyl-carbinol herzustellen.

Zum einen sind chemische Synthesen bekannt.

Die auf chemischer asymmetrischer Synthese beruhenden Methoden zur Herstellung von (S)-PAC, generieren einen ee von 68 % bzw. 86%. Die Verfahren sind in den Veröffentlichungen von Davis, Franklin A.; Sheppard, Aurelia C.; Lal, G. Sankar Tetrahedron Letters, 1989, vol. 30, 7 p. 779 - 782 und Adam, Waldemar; Fell, Rainer T.; Stegmann, Veit R.; Saha-Moeller, Chantu R. Journal of the American Chemical Society, 1998, vol. 120, 4 p. 708 - 714 beschrieben. Zudem gibt es Methoden, bei denen (*S*)-PAC nur als Nebenprodukt entsteht und (*R*)-PAC im Enantiomerenüberschuss vorliegt wie z.B. bei folgenden Reaktionen, wie der Reduktion von 1-Phenylpropan-1,2-dion, die in den Veröffentlichungen von Toukoniitty, Esa; Maeki-Arvela, Paeivi; Kuzma, Marek; Villela, Alexandre; Kalantar Neyestanaki, Ahmad; Salmi, Tapio; Sjoeholm, Rainer; Leino, Reko; Laine, Ensio; Murzin, Dmitry Yu, Journal of Catalysis, 2001, vol. 204, 2 p. 281 - 291 und der Synthese ausgehend von Benzaldehyd die von Fleming, Steven A.; Carroll, Sean M.; Hirschi, Jennifer; Liu, Renmao; Pace, J. Lee; Redd, J. Ty Tetrahedron Letters, 2004, vol. 45, 17 p. 3341 - 3343 und der Reaktion von 2-Hydroxy-2-Phenylacetonitril von Brussee, J.; Roos, E. C.; Gen, A. Van Der Tetrahedron Letters, 1988, vol. 29, 35 p. 4485 - 4488 beschrieben sind.

Darüber hinaus ist eine Synthese beschrieben, bei der der chirale Baustein, 1-Phenylpropan-1,2-diol, zu (S)-PAC oxidiert werden kann. (*S*)-PAC entsteht mit einem Enantiomerenüberschuss (ee) von 91 %, wie von Zi-Qiang Rong, Hui-Jie Pan, Hai-Long Yan, und Yu Zhao Organic Letters, 2014, 16 (1), pp 208-211 beschrieben, bzw. 69 % wie von Waldemar Adam, Chantu R. Saha-Möller, und Cong-Gui Zhao Journal of Organic Chemistry, 64(20), 7492-7497; 1999 beschrieben wurde, ist aber zudem mit einem Regioisomer verunreinigt, das aufwändig abgetrennt werden muss.

Weiterhin ist eine emzymatische asymmetrische Synthese bekannt die in der Dissertation von Álvaro Gómez Baraibar mit dem Titel "Development of a biocatalytic production process for (S)-alpha-hydroxy ketones" aus dem Jahr 2013 beschrieben ist. Verwendet man dieses Enzym gemäß dieser Dissertation heterolog in *Escherichia coli* exprimert für die Synthese in ganzen Zellen, beträgt die optische Reinheit von (S)-PAC ∼43 % ee.

Diese einzige enzymatische, asymmetrische Synthese von (*S*)-PAC wurde in einer Carboligationsreaktion ausgehend von Benzaldehyd und Acetaldehyd, bzw. Benzaldehyd und Pyruvat beschrieben. Die Reaktion wird von einer Variante des Enzyms Pyruvatdecarboxylase aus *Acetobacter pasteurianus*, der ApPDC-E469G, katalysiert, bei der in Position 469 Glutamat durch Glycin ausgetauscht ist. Der höchste Enantiomerenüberschuss, der dabei mit dem isolierten Enzym erreicht wurde, liegt bei 89 %, wie es von Rother Nee Gocke, Doerte; Kolter, Geraldine; Gerhards, Tina; Berthold, Catrine L.; Gauchenova, Ekaterina; Knoll, Michael; Pleiss, Juergen; Mueller, Michael; Schneider, Gunter; Pohl, Martina der Veröffentlichung in ChemCatChem, 2011, vol. 3, 10 p. 1587 - 1596 beschrieben wird.

Ausgehend von dem Stand der Technik ist es die Aufgabe der Erfindung ein enzymatisches Verfahren zur asymmetrischen Synthese von (*S*)-Phenylacetylcarbinol zur Verfügung zu stellen, das einen höheren Enantiomerenüberschuss von (*S*)-Phenylacetylcarbinol ermöglicht. Der Enantiomerenüberschuss von (*S*)-Phenylacetylcarbinol soll höher als 89% sein. Bei einer Herstellung mit ganzen Zellen sollte der Enantiomerenüberschuss größer als 43% sein. Weiterhin sollen keine Nebenprodukte und keine Regioisomere gebildet werden. Dabei sollen preisgünstige Edukte eingesetzt werden können, die nicht chiral sind. Es soll eine asymmetrische Synthese von (*S*)-Phenylacetylcarbinol ermöglicht werden. Teure Trennung von chiralen Produktgemischen sollen verhindert werden.

Es soll ein Enzym bereitgestellt werden, mit dem (*S*)-Phenylacetylcarbinol aus Benzaldehyd und Pyruvat oder Acetaldehyd hergestellt werden kann sowie eine für das Enzym kodiernde DNA und einen Vektor, enthaltend die DNA.

Weiterhin soll ein Verfahren zur Herstellung des Enzyms zur Verfügung gestellt werden. Die nach dem Stand der Technik vorliegenden Nachteile und Probleme sollen überwunden werden.

Es soll ein Verfahren zur Herstellung von (*S*)-Phenylacetylcarbinol zur Verfügung gestellt werden, das auch bei Einsatz von Rohzellextrakten oder ganzen Zellen hohe Enantiomerenüberschüsse ermöglicht.

Die Nachteile des Standes der Technik sollen kumulativ überwunden werden.

Ausgehend vom Oberbegriff des Anspruchs 1 und der nebengeordneten Ansprüche wird die Aufgabe erfindungsgemäß gelöst mit den im kennzeichnenden Teil der Ansprüche angegebenen Merkmalen. Die Aufgabe wird erfindungsgemäß gelöst durch eine Variante der Lyase ApPDC-E469G bei der das Tryptophan in Position 543 durch eine Aminosäure ausgetauscht ist, die sterisch kleiner, bzw. eine gegenüber Tryptophan verminderte Raumerfüllung hat, sowie dafür kodierende Desoxyribonukleinsäuren und Vektoren, die diese Desoxyribonukleinsäuren enthalten. Weiterhin wird die Aufgabe dadurch gelöst, dass diese Lyasen nach dem nebengeordneten Anspruch für die Umsetzung von Benzaldehyd mit Pyruvat oder Acetaldehyd zu (*S*)-Phenylacetylcarbinol eingesetzt werden.

Mit den erfindungsgemäßen Lyasen, der dafür kodierenden DNA, dem Vektor, sowie dem Herstellungsverfahren für (*S*)-Phenylacetylcarbinol ist es nunmehr möglich, (*S*)-Phenylacetylcarbinol bei Verwendung des isolierten Enzyms in einem Enantiomerenüberschuss von 97% ee, bei Verwendung von ganzen Zellen von 95% ee und bei Verwendung eines Rohzellextraks von 94% ee herzustellen. Es werden keine Nebenprodukte, insbesondere keine Regioisomere gebildet. Dadurch, dass die Synthese mit unchiralen Edukten auskommt, ist sie preisgünstig. Auf eine Enantiomerentrennung kann verzichtet werden. Bei der Herstellung von (*S*)-Phenylacetylcarbinol können auch mit Rohzellextrakten oder ganzen Zellen hohe Enantiomerenüberschüsse erreicht werden. Alle nach dem Stand der Technik beschriebenen Nachteile werden kumulativ ausgeräumt.

Vorteilhafte Weiterbildungen der Erfindung sind in den Unteransprüchen angegeben.

Erfindungsgemäß wird eine Lyase zur Verfügung gestellt, bei der Trypotophan in dem gegenüber dem Wildtyp aus *Aceobacter pasteurianus* veränderten Protein ApPDC-E469G in Position 543 durch eine Aminosäure ersetzt ist, die sterisch kleiner als Tryptophan, bzw. weniger raumerfüllend als Tryptophan ist.

Diese Lyase hat einen positiven Einfluss auf die Erhöhung der Stereoselektivität bei der Herstellung von (*S*)-Phenylacetylcarbinol.

Vorzugsweise können folgende Lyasen genannt werden, die diese Anforderung erfüllen:
- ApPDC-E469G-W543H gemäß Sequenz Nr. 1 mit Histidin in Position 543
- ApPDC-E469G-W543F gemäß Sequenz Nr. 3 mit Phenylalanin in Position 543
- ApPDC-E469G-W543P gemäß Sequenz Nr. 5 mit Prolin in Position Nr. 543
- ApPDC-E469G-W543I gemäß Sequenz Nr. 7 mit Isoleucin in Position Nr. 543
- ApPDC-E469G-W543L gemäß Sequenz Nr. 9 mit Leucin in Position Nr. 543
- ApPDC-E469G-W543M gemäß Sequenz Nr. 11 mit Methionin in Position Nr. 543
- ApPDC-E469G-W543V gemäß Sequenz Nr. 13 mit Valin in Position 543
- ApPDC-E469G-W543A gemäß Sequenz Nr. 15 mit Alanin in Position Nr. 543
- ApPDC-E469G-W543Y gemäß Sequenz Nr. 17 mit Tyrosin in Position Nr. 543
- ApPDC-E469G-W543T gemäß Sequenz Nr. 19 mit Threonin in Position Nr. 543
- ApPDC-E469G-W543G gemäß Sequenz Nr. 21 mit Glycin in Position Nr. 543
- ApPDC-E469G-W543S gemäß Sequenz Nr. 23 mit Serin in Position Nr. 543
- ApPDC-E469G-W543C gemäß Sequenz Nr. 25 mit Cystein in Position Nr. 543

Weiterhin werden erfindungsgemäß Desoxyribonukleinsäuren zur Verfügung gestellt, die für die angegebenen Enzyme kodieren.

Erfindungsgemäß handelt es sich um, Desoxyribonukleinsäuren, die für eine Variante des Enzyms ApPDC-E469G kodieren die in Position 1627-1629 für eine Aminosäure kodieren, die gegenüber Tryptophan eine verminderte Raumerfüllung aufweist.

Vorzugsweise kodiert die Desoxyribonukleinsäure für die Proteine nach den Sequenzen 1, 3, 5, 7, 9, 11, 13, 15, 17, 19, 21, 23 und 25.

Beispielhaft können folgende Desoxyribonukleinsäuren genannt werden.
- Sequenz Nr.2 kodierend für ApPDC-E469G-W543H
- Sequenz Nr.4 kodierend für ApPDC-E469G-W543F
- Sequenz Nr.6 kodierend für ApPDC-E469G-W543P
- Sequenz Nr.8 kodierend für ApPDC-E469G-W543I
- Sequenz Nr.10 kodierend für ApPDC-E469G-W543L
- Sequenz Nr.12 kodierend für ApPDC-E469G-W543M
- Sequenz Nr.14 kodierend für ApPDC-E469G-W543V
- Sequenz Nr.16 kodierend für ApPDC-E469G-W543A
- Sequenz Nr.18 kodierend für ApPDC-E469G-W543Y
- Sequenz Nr.20 kodierend für ApPDC-E469G-W543T
- Sequenz Nr.22 kodierend für ApPDC-E469G-W543G
- Sequenz Nr.24 kodierend für ApPDC-E469G-W543S
- Sequenz Nr.26 kodierend für ApPDC-E469G-W543C

Für das Beispiel nach Sequenz Nr.2, bei dem in dieser Position für die Aminosäure Histidin kodiert wird, können sich in den Positionen 1627-1629 beispielsweise die Nukleinsäuren TGG befinden.

In einer Ausführungsform der Erfindung sind die Desoxyribonukleinsäuren in einen Vektor, vorzugsweise ein Plasmid ligiert.

Als Leervektoren können beispielsweise pET-20b(+),pET-21a-d(+), pET-22b(+),pET-23a-d(+),pET-24a-d(+),pET-25b(+), pET-26b(+), pET-27b(+), pET-28a(+), pET-29a-c(+), pET-30a-c(+), pET-31b(+), pET-34b(+),pET-35b(+),pET-36b(+),pET-37b(+),pET-38b(+) eingesetzt werden, in die die entsprechende erfindungsgemäße DNA ligiert wird.

Alternativ können die Desoxyribonukleinsäuren auch in das Genom ligiert sein.

Bei den ligierten Desoxyribonukleinsäuren handelt es sich um eine DNA-Sequenz, die für eine Variante des Enzyms ApPDC-E469G kodiert und die in Position 1627-1629 für eine Aminosäure kodiert, die gegenüber Tryptophan eine verminderte Raumerfüllung aufweist.

Vorzugsweise kodiert die ligierte Desoxyribonukleinsäure für die Proteine nach den Sequenzen 1, 3, 5, 7, 9, 11, 13, 15, 17, 19, 21, 23 und 25.

Beispielhaft können Desoxyribonukleinsäuren nach den Sequenzen Nr. 2, 4, 6, 8, 10, 12, 14, 16, 18, 20, 22, 24, 24 und 26 genannt werden.

Erfindungsgemäß werden Vektoren zur Verfügung gestellt, welche eine Desoxyribonukleinsäure enthalten, die für eine Variante des Enzyms ApPDC-E469G kodiert und die in Position 1627-1629 für eine Aminosäure kodiert, die gegenüber Tryptophan eine verminderte Raumerfüllung aufweist.

Vorzugsweise enthält der Vektor eine Desoxyribonukleinsäure nach einer der Sequenzen Nr. 2, 4, 6, 8, 10, 12, 14, 16, 18, 20, 22, 24, 24 oder 26.

Vorzugsweise ist der Vektor ein Plasmid.

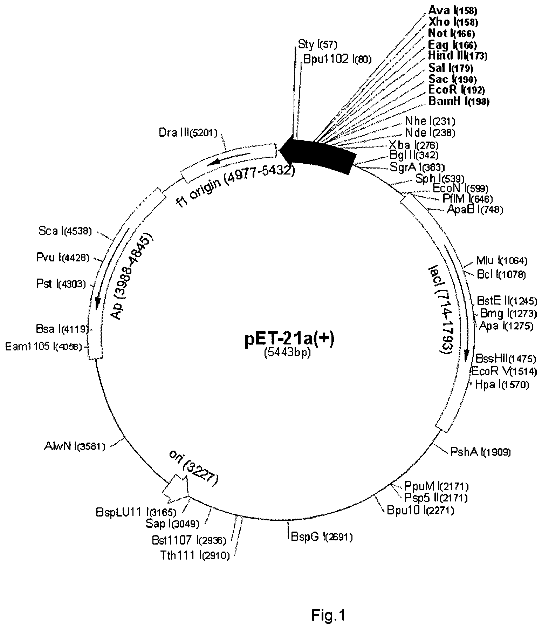

Figur 1 zeigt ein erfindungsgemäßes Plasmid (pET21 a(+) Vektorkarte).

In Sequenz Nr.27 ist beispielhaft eine DNA-Sequenz für ein erfindungsgemäßes Plasmid dargestellt, die eine DNA nach Sequenz Nr. 2 enthält.

Eine für die erfindungsgemäßen Enzyme kodierende DNA kann nach dem Fachmann bekannten Verfahren durch gerichtete oder ungerichtete Mutagenese hergestellt werden. Bevorzugt ist dabei die gerichtete Mutagenese. Diese Verfahren sind dem Fachmann bekannt. Im speziellen Beschreibungsteil wird ein Beispiel für eine Herstellung für eine Ausführungsform der Erfindung konkret offenbart. Diese Verfahrensweise kann grundsätzlich auch für alle anderen offenbarten Desoxyribonukleinsäuren und Enzyme eingesetzt werden, so dass alle erfindungsgemäßen Enzyme und Desoxyribonukleinsäuren auf analogem Weg hergestellt werden können.

Erfindungsgemäß wird Benzaldehyd mit Pyruvat oder mit Acetaldehyd gemäß Formel (1) mittels einer Variante des Enzyms ApPDC-E469G, die in Position 543 eine Aminosäure aufweist, die gegenüber Tryptophan eine verminderte Raumerfüllung aufweist, vorzugsweise ein Enzym aus der Gruppe nach Sequenz Nr. 1, 3, 5, 7, 9, 11, 13, 15, 17, 19, 21, 23 oder 25 zu (S)-Phenylacetylcarbinol umgesetzt.

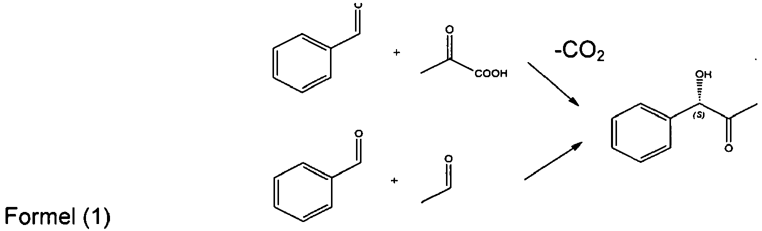

Die Umsetzung erfolgt vorzugsweise in wässriger Lösung.

Der pH-Wert liegt in einem Bereich von 5-9, vorzugsweise 6,5 - 8, besonders bevorzugt 6,5 -7.

Dazu können als Puffer beispielsweise Kaliumphosphatpuffer, HEPES, MOPS, TEA oder TRIS-HCl eingesetzt werden.

Weiterhin können als Kofaktoren Thiamindiphosphat und Magnesiumsulfat eingesetzt werden.

Für die Produktion von (*S*)-Phenylacetylcarbinol kann als Produktionsorganismus beispielsweise *E.coli, ein Corynebakterium,* beispielsweise *Corynebakterium glutamicum,* oder eine Hefe, wie *Saccheromycies cerevisiae* eingesetzt werden.

Dazu werden die Produktionsorganismen mit der erfindungsgemäßen DNA oder einem Vektor, der die DNA enthält transformiert.

Die DNA kann in dem Produktionsorganismus auch in das Genom eingeführt sein.

Die erfindungsgemäß eingesetzten Gene werden dabei heterolog exprimiert.

Für die *in vitro* Produktion kann entweder das isolierte Enzym oder der Zellextrakt des Produktionsorganismus eingesetzt werden.

Typische Temperaturen liegen zwischen 20°C und 40°C, bevorzugt sind 20°C bis 30°C, besonders bevorzugt ist eine Temperatur von 20°C bis 25°C.

Die Reaktionszeiten können 2h - 48 h, vorzugsweise 6h - 24h, besonders bevorzugt 12 h sein.

Im Folgenden werden einige Beispiele vorgestellt, die nicht beschränkend auszulegen sind.

Die Reaktionen können in einem klassischen Ansatz in einem Reaktionskolben unter Rühren durchgeführt werden.

### Ausführungsbeispiel 1:

20 mM Benzaldehyd, 400 mM Pyruvat, 2,5 mM Magnesiumsulfat, 100 µM Thiamindiphosphat, 5 mg/mL ApPDC-E469G-W543H (Rohzellextrakt von E. coli Zellen in denen ApPDC-E469G-W543H exprimiert vorlag), 50 mM Kaliumphosphat-Puffer pH 6.5, 25 °C, Reaktionszeit: 48 h.
Enantiomerenreinheit von (S)-PAC: ee 94 %

### Ausführungsbeispiel 2:

20 mM Benzaldehyd, 400 mM Pyruvat, 2,5 mM Magnesiumsulfat, 100 µM Thiamindiphosphat, 20 mg/mL (Feuchtgewicht) ApPDC-E469G-W543H (ganze Zellen von E. coli in denen ApPDC-E469G-W543H exprimiert), 50 mM Kaliumphosphat-Puffer pH 6.5, 25 °C, Reaktionszeit: 48 h
Enantiomerenreinheit von (S)-PAC: ee 95 %
Ausbeute: 68%

### Ausführungsbeispiel 3:

20 mM Benzaldehyd, 400 mM Pyruvat, 2,5 mM Magnesiumsulfat, 100 µM Thiamindiphosphat, 1 mg/mL ApPDC-E469G-W543H (isoliertes Enzym), 50 mM Kaliumphosphat-Puffer pH 6.5, 25 °C, Reaktionszeit: 48 h.
Enantiomerenreinheit von (S)-PAC: ee 97 %
Ausbeute: 64%

Die Variante, ApPDC-E469G-W543H produziert (S)-PAC unter Verwendung von isolierten Enzymen mit einem ee von 97 %. Unter Verwendung von ApPDC-E469G-W543H, das heterolog in Escherichia coli exprimiert wurde und als kostengünstiger Ganzzellen-Katalysator eingesetzt wird, kann (*S*)-PAC mit einem ee von 95 % generiert werden. Die Verwendung als Zellextrakt mit heterolog in E. coli exprimierter ApPDC-E469G-W543H Variante führt zu einem ee von 94 %.

Im Folgenden wird die Herstellung der Desoxyribonukliensäure, die für das Enzym ApPDC-E469G-W543H kodiert, sowie des Enzyms genauer beschrieben.

Die Methode des sogenannten "Site saturated mutagenesis" nach der Variante von Reetz *et. al* (M. T. Reetz, D. Kahakeaw and R. Lohmer, ChemBioChem, 2008 (9) 1797-1804) wurde ausgehend von der Gensequenz *Ap*PDC-E469G (Template-DNA) durchgeführt, um Aminosäureaustausche an der Position W543 zu erhalten. Bei dieser Methode werden NDT-Kodons verwendet, die für 12 von 20 natürlichen Aminosäuren kodieren.

### Polymerasekettenreaktion (PCR)

In einem initialen Schritt wird die Template-DNA mittels Polymerasekettenreaktion (PCR) vervielfältigt und dabei gleichzeitig durch die Verwendung von sogenannten degenerierten Primer und den NDT-Kodons Mutationen eingefügt. Die verwendeten Primer wurden von der Firma "Eurofins MWG Operon" (http://www.eurofinsgenomics.eu) bestellt und hatten folgende Sequenz:
Primer für die Site Saturated Mutagenesis zur Herstellung von ApPDC-E469G-W543NDT
vorwärts: 5'GGATATGCTGGTTCAANDTGGCCGCAAGGTTGC 3 (Seq. Nr.30)
rückwärts: 5'GGCAACCTTGCGGCCAHNTTGAACCAGCATATC 3' (Seq. Nr.31)

Es wurde zunächst eine Master-Lösung hergestellt und anschließend in vier Ansätze zu je 50 µl aufgeteilt. Zur Start der Reaktion wurde 1 µl "KOD Hot Start Polymerase" hinzugegeben.

### PCR-Reaktionsansatz:

1-fach PCR-Puffer
5 % (v/v) DMSO
2 mM MgSO₄
0.2 mM Nukleotide
0.25 pmol vorwärts Primer
0.25 pmol rückwärts Primer
0.1 ng/µl DNA-Template

Die Reaktion wurde unter folgenden Bedingungen durchgeführt:

| | Dauer (min) | Temperatur (°C) | Wiederholungen |
|---|---|---|---|
| Initialisierung | 2:00 | 95 | 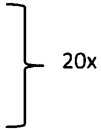 |
| Denaturierung | 0:20 | 95 | |
| *Annealing* | 1:00 | 75.5 °C | |
| Elongation | 6:00 | 70 | |
| Termination | 10:00 | 70 | |

Zum Verdau der Template-DNA wurde 1 µl des Enzyms Dpnl (Eppendorf) zur Lösung hinzugegeben und für 1 h bei 37°C inkubiert. Der gesamte Ansatz wurde dann mit dem "DNA Purification Kit" (Chemikalienliste) vor der weiteren Transformation gereinigt.

### Transformation von E.coli BL21-DE3 und E.coli DH5α

Die Stämme *E.coli* BL21-DE3 und *E.coli* DH5a wurden mit der durch "Site Saturation Mutagenesis" hergestellte DNA transformiert. Dazu wurde zu 50 µl kompetenten Zellen 100 ng der DNA hinzugegeben uns auf Eis für 30 min inkubiert. Anschließend erfolgte ein Hitzeschock für 90 sek. bei 42 °C. Nach 3 min. auf Eis wurden 500 µl SOC-medium hinzugegeben und die Lösung am Anschluss für 45 min bei 350 UPM und 37 °C im Eppendorf Thermomixer inkubiert. Nach erfolgter Inkubation wurde die Zellsuspension bei 13.000 UPM in einer Eppendorf Tischzentrifuge für 30 sek zentrifugiert und das Pellet daraufhin in 100 µl Überstand resuspendiert. Die auf 100 µl eingeengte Zellsuspension wurde auf LB-Aggarplatten (mit 100 µg/ml Ampicillin) ausplattiert und für 16 h bei 37 °C über Kopf inkubiert.

### Expression der Enzymvarianten

46 Einzelkolonien der Transformation wurden von der Platte mit einem Zahnstocher gepickt und für 24 h bei 20 °C und 850 UPM in jeweils einem *well* einer 48er-Nerbe-Platte (Nerbe Plus GmbH) mit je 1 ml LB-Medium inkubiert ("Masterplate"). Ein weiteres *well* wurde mit *E.coli* BL 21-DE3 Zellen inokuliert, die zuvor analog mit der ApPDC-E469G-Template-DNA transformiert wurden. Nach erfolgter Inkubation wurden zu je 1.5 ml Autoinduktionsmedium 10 µl der Zellsuspensionen in 48-well-FlowerPlates® (m2p-labs, Germany) gegeben. Die FlowerPlate wurde für 48 h bei 20 °C und 850 UPM inkubiert. Das restliche Volumen (990 µl) der "Masterplate" wurde mit 300 µl Glycerin versetzt und bei -80 °C gelagert.

### Zellaufschluss und Carboligation

Die in den FlowerPlates® (blumenartige Schikanen in einer 48-well-Platte) exprimierten Varianten wurden eingefroren (48 h, 4°C). Nach dem Wiederauftauen wurden je 500 µl der Zellsuspensionen in zwei *wells* einer 96-*well*-Platte überführt (Doppelbestimmung). Die Platte wurde für 3 min bei 4.000 UPM zentrifugiert und das Pellet in 420 µl KPi-Puffer mit 1 mg/ml Lysozym resuspendiert. Die Platte wurde für 1 h bei 20 °C und 400 UPM inkubiert und anschließend erneut für 10 min bei 4.000 UPM zentrifugiert. Jeweils 250 µl des Überstands wurde in jeweils ein *well* einer 2 ml-Nerbe-Platte pipettiert und 250 µl einer Reaktionslösung aus 40 mM Benzaldehyd, 400 mM Pyruvat, 4 mM Magnesiumsulfat und 400 µM Thiamindiphosphat hinzugegeben. Die Platte wurde erneut für 24 h inkubiert und die Reaktionslösungen anschließend analysiert (siehe HPLC-Analyse).

### HPLC-Analyse

200 µl der Carboligation-Reaktionslösungen wurden mit jeweils 200 µl Heptan versetzt, gevortext und anschließend jeweils 150 µl der oberen Phase in HPLC-Vials überführt. Die Analyse der Proben erfolgte mit einer Chiralpak IC-3 Säule (Chiral Technologies Inc.)unter Verwendung der folgenden Methode.

**HPLC Programm**

| | |
|---|---|
| Länge | 24 min |
| Fluss | 0.5 ml/min |
| mobile Phase | 25 % Isopropanol |
| | 75 % Heptan |

**Typische Retentionszeiten und verwendete Wellenlänge zur Quantifizierung**

| | Retentionszeit (min) | Wellenlänge (nm) |
|---|---|---|
| (*R*)-PAC | 12.3 | 210 |
| (*S*)-PAC | 12.9 | 210 |
| Benzaldehyd | 11.4 | 254 |

### DNA-Isolierung und Identifizierung der besten Enzymvarianten durch DNA-Sequenzierung

Die DNA des Enzyms, das in den Carboligationsreaktionen den höchsten ee-Werten für (S)-PAC lieferte, wurde ausgehend von der Masterplatte zur Identifizierung der Mutation sequenziert. Dazu wurden zunächst Zellen mit einer Impföse aus der mit Glycerin versetzten Masterplatte 50 mL LB-Medium (+50 µg/ml Ampicillin) überführt und in einem 250 mL Erlenmeyerkolben bei 37 °C inkubiert. Nach 12 h Inkubation wurden 20 mL der Zellsuspension zentrifugiert (4.000 UPM, 5 min, 4 °C). Die DNA der Zellen im Pellet wurde analog zu den Herstellerangaben (Qiagen N.V.) nach der Methode des "QIAprep® Spin Miniprep Kit" isoliert. Die Konzentration der DNA wurde dazu auf 100 ng/µl eingestellt und durch die Firma LGC Genomics GmbH sequenziert.

**LB (Lysogeny Broth)-Medium**

| | |
|---|---|
| 10 g/l | NaCl |
| 10 g/l | Peptone |
| 5 g/l | Yeast extract |

### Alternative, gerichtete Methode zur Herstellung der Variante ApPDC-E469G-W543H mittels QuikChange®

Eine andere Methode zur Herstellung der Enzymvariante *Ap*PDC-E469G/W543H ist beispielsweise die sogenannte QuikChange®-PCR-Methode (U.S. Patent Nr. 5.789.166, 5.932.419, 6.391.548). Bei dieser Variante der PCR wird ein Primerpaar verwendet, das an der Stelle des auszutauschenden DNA-Triplettcodes die entsprechende Sequenzveränderung trägt. Zur Herstellung der Enzymvariante *Ap*PDC-E469G/W543H kann das Gen verwendet werden, das für die Variante ApPDC-E469G kodiert. Dieses so genannte DNA-Template sollte in einem Vektor (beispielsweise pET22a) kloniert vorliegen. Anstelle des Triplett-Codes, der in Position W543 für die Aminosäure Tryptophan kodiert, muss ein Primer verwendet werden, der die für Histidin kodierende Mutation an dieser Position trägt (also: CAC oder CAT). Alle weiteren Parameter dieser QuikChange®-PCR-Methode sowie die Auswahl der benötigten Primer können analog zu den Angaben des Herstellers (Agilent Technologies, Inc.) mittels der Anleitung des "QuikChange® Site-Directed Mutagenesis Kit" durchgeführt werden.

### DNA-Template (ApPDC-E469G) der QuikChange®-PCR-Methode zur Herstellung der Variante ApPDC-E469G-W543H

Die Sequenz ist im Sequenzprotokoll als Seq. Nr. 28 bezeichnet.

Sequenz Nr. 28 ist hier beispielhaft für eine DNA offenbart, die für das zu verändernde Protein nach Sequenz Nr. 29 kodiert. Erfindungsgemäß können jedoch alle anderen für das zu verändernde Ausgangsprotein kodierenden Desoxyribonukleinsäuren für die Herstellung des zu verändernden Enzyms eingesetzt werden. Die dafür kodierenden Nukleotide sind dem Fachmann bekannt.

Die zugehörige Proteinsequenz ist im Sequenzprotokoll als Seq. Nr. 29 bezeichnet.

### Herstellung der Varianten in Form von "ganzen Zellen"

Zur Expression der Enzyme in ganzen Zellen im 1L-Maßstab wurde zunächst Zellen von der mit Glycerin versetzten Masterplatte mit einer Impföse in 50 mL LB-Medium (+100 µg/ml Ampicillin) überführt und in einem 250 mL Erlenmeyerkolben bei 120 UPM und 37 °C inkubiert. Nach 16 h Inkubation wurden die zu 1 L Autoinduktionsmedium 10 mL der Kultur gegeben und in einem 5 L Erlenmeyerkolben für 72 h bei 90 UPM und 20 °C inkubiert. Die Zellen wurden anschließend durch Zentrifugation (4 °C, 6.000 UPM, 30 min) geerntet und bis zur weiteren Verwendung bei -20 °C gelagert.

**Autoinduktionsmedium**

| | |
|---|---|
| 12 g/l | Pepton |
| 24 g/l | Hefeextrakt |
| 90 mM | Kaliumphosphat-Puffer (pH 7,5) |
| 0,5 g/l | Glucose |
| 2 g/l | Lactose |
| 0,01 g/l | Ampicillin |
| 6.3 g/l | Glycerin |

### Herstellung der Varianten in Form von isolierten Enzymen

10 g der im 1L-Maßstab kultivierten Zellen wurden mit 25 mL Aufschlusspuffer (50 mM Kaliumphosphat pH 6.5, 100 µM Thiamindiphosphat, 2 mM Magnesiumsulfat), der auf 4 °C gekühlt war, auf Eis resuspendiert. Anschließend wurden die resuspendierten Zellen mittels Ultraschall (SD14-Sonotrode (Hielscher Ultrasonics GmbH), 4x2 min Beschall mit je 1 min Abkühlung aus Eis) aufgeschlossen. Zur Abtrennung von Zelltrümmern wurde die Lösung zentrifugiert (45 min, 18.000 UPM, 4 °C) und der Überstand ("Zellextrakt") in ein neues Gefäß überführt.

Zur Reinigung der *Ap*PDC-Variante mittels immobilisierter Metallionen-Affinitätschromatographie und Größenausschlusschromatographie wurde ein "ÄKTA™purifier" der Firma "Amersham Bioscience" verwendet, um u.a. die Protein-UV-Absorption (280 nm) sowie die elektrische Leitfähigkeit zu detektieren und die Flussrate einzustellen. Zur Reinigung wurde der hergestellte Zellextrakt (∼25 mL) mit einem Flussrate von 3 mL/min auf eine Säule mit einem Volumen von 60 mL Ni-NTA-Superflow (Qiagen N.V.) aufgetragen, die zuvor mit 180 mL des Auftragspuffer equilibriert wurde. Im Anschluss wurde weiter mit Auftragspuffer in einer Flussrate von 5 ml/min gespült, um nicht, bzw. sehr schwach an das Säulenmaterial bindende Proteine zu entfernen. Nachdem die UV-Absorption (280 nm) wieder eine stabile Basisline erreicht hatte, wurde ein Waschpuffer (50 mM Kaliumphosphat pH 6.5, 100 µM Thiamindiphosphat, 2 mM Magnesiumsulfat, 50 mM Imidazol) mit einer Flussrate von 5 mL/min zur Elution von schwach an das Säulenmaterial bindenden Proteinen verwendet. Nach erneut stabiler UV-Absorption (280 nm) wurde zur Elution des Zielproteins ein Elutionspuffer (50 mM Kaliumphosphat pH 6.5, 100 µM Thiamindiphosphat, 2 mM Magnesiumsulfat, 250 mM Imidazol) mit einer Flussrate von 5 mL/min verwendet.

Das Eluat der IMAC wurde zur Umpufferung mit einer Flussrate von 10 mL/min auf eine Größenausschlusschromatographie-Säule (1 L Säulenvolumen, Sephadex-G25, GE-Healthcare) aufgetragen, die zuvor mit 2 L Umpufferungspuffer (10 mM Kaliumphosphat pH 6.5, 100 µM Thiamindiphosphat, 2 mM Magnesiumsulfat) gespült wurde. Die Fraktionen mit erhöhter UV-Absorption (280 nm) wurden vereinigt und in einer Kristallisationsschale eingefroren (-20 °C). Zur Gefriertrocknung wurde an die eingefrorene Proteinlösung für 3 Tage ein Unterdruck von 0.22 mBar angelegt. Das entstandene Pulver hatte einen Proteingehalt von 20 %. Die Reinheit (Anteil des Zielproteins in Bezug auf Fremdproteine) betrug >90 %.

## Patentansprüche

1. Lyase,
**dadurch gekennzeichnet,**
**dass** sie eine Aminosäuresequenz nach einer der Sequenzen 1, 3, 9 oder 21 hat.

2. Desoxyribonukleinsäure, kodierend für eine Lyase,
**dadurch gekennzeichnet,**
**dass** sie eine Nukleotidsequenz nach den Sequenzen Nr. 2, 4, 10 oder 22 hat.

3. Vektor,
**dadurch gekennzeichnet,**
**dass** er eine Sequenz nach Anspruch 2 beinhaltet.

4. Vektor nach Anspruch 3,
**dadurch gekennzeichnet,**
**dass** er ein Plasmid ist.

5. Vektor nach Anspruch 4,
**dadurch gekennzeichnet,**
**dass** die Sequenzen nach Anspruch 2 in einem Leervektor aus der Gruppe pET-20b(+),pET-21a-d(+), pET-22b(+),pET-23a-d(+),pET-24a-d(+),pET-25b(+), pET-26b(+), pET-27b(+), pET-28a(+), pET-29a-c(+), pET-30a-c(+), pET-31b(+), pET-34b(+),pET-35b(+),pET-36b(+),pET-37b(+),pET-38b(+) ligiert sind.

6. Verfahren zur Herstellung von (S)-Phenylacetylcarbinol, bei dem Benzaldehyd mit Pyruvat oder mit Acetaldehyd nach Formel (1) umgesetzt wird, 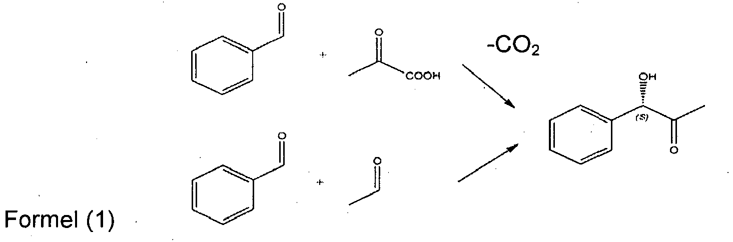 **dadurch gekennzeichnet,**
**dass** die Umsetzung mit einer Lyase nach Anspruch 1 durchgeführt wird.

7. Verfahren nach Anspruch 6,
**dadurch gekennzeichnet,**
**dass** die Produktion von (*S*)-Phenylacetylcarbinol in einem Produktionsorganismus aus der Gruppe von *E.coli, ein Corynebakterium,* beispielsweise *Corynebakterium glutamicum,* oder eine Hefe, wie *Saccheromycies cerevisiae* durchgeführt wird.

8. Verfahren nach einem der Ansprüche 6 oder 7,
**dadurch gekennzeichnet,**
**dass** die Organismen nach Anspruch 7 mit mindestens einer DNA der Sequenzen 2, 4, 10 oder 22 transformiert werden und/oder die DNA in das Genom integriert wird.

9. Verfahren nach Anspruch 8,
**dadurch gekennzeichnet,**
**dass** für die Transformation ein Vektor nach einem der Ansprüche 3 bis 5 eingesetzt wird.

10. Verfahren nach Anspruch 6,
**dadurch gekennzeichnet,**
**dass** die Umsetzung *in vitro* durchgeführt wird.

11. Verfahren nach Anspruch 10,
**dadurch gekennzeichnet,**
**dass** das isolierte Enzym nach einer der Sequenzen 1, 3, 9 oder 21 eingesetzt wird.

12. Verfahren nach Anspruch 10,
**dadurch gekennzeichnet,**
**dass** ein Rohzellextrakt aus einem Produktionsorganismus verwendet wird.

## Claims

1. Lyase,
**characterised in that**
it has an amino acid sequence according to one of sequences 1, 3, 9 or 21.

2. Deoxyribonucleic acid, coding for a lyase,
**characterised in that**
it has a nucleotide sequence according to sequences No. 2, 4, 10 or 22.

3. Vector,
**characterised in that**
it contains a sequence according to claim 2.

4. Vector according to claim
3,
**characterised in that**
it is a plasmid.

5. Vector according to claim 4,
U:\01_Patente\2666\PCT\EP\20190626 1.2666PCTEP_tansl patent claims.docxthe sequences according to claim 2 are ligated in an empty vector from the group pET- 20b(+), pET-21a-d(+), pET-22b(+), pET-23a-d(+), pET-24a-d(+), pET-25b(+), pET-26b(+), pET-27b(+), pET-28a(+), pET-29a-c(+), pET-30a-c(+), pET-31b(+), pET-34b(+), pET-35b(+), pET-36b(+), pET-37b(+), pET-38b(+).

6. Method for producing (S)-phenylacetylcarbinol, in which benzaldehyde is converted with pyruvate or with acetaldehyde according to formula (1), 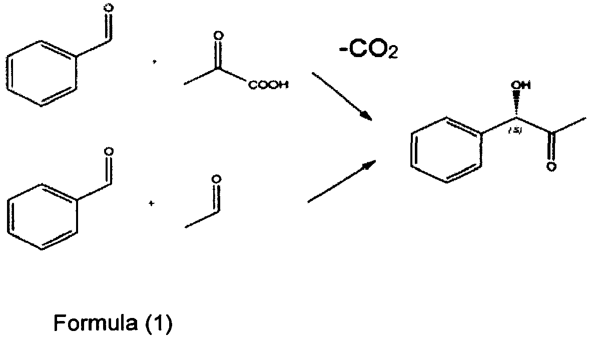 **characterised in that** the conversion is carried out with a lyase according to claim 1.

7. Method according to claim 6,
**characterised in that**
the production of (S)-phenylacetylcarbinol is carried out in a production organism from the group of *E.coli, a corynebacterium, corynebacterium glutamicum* for example, or a yeast, such as *saccharomyces cerevisiae.*

8. Method according to one of claims 6 or 7,
**characterised in that**
the organisms according to claim 7 are transformed with at least one DNA of sequences 2, 4, 10 or 22 and/or the DNA is integrated into the genome.

9. Method according to claim 8,
**characterised in that**
a vector according to one of claims 3 to 5 is used for the transformation.

10. Method according to claim 6,
**characterised in that**
the conversion is carried out *in vitro.*

11. Method according to claim 10,
**characterised in that**
the isolated enzyme according to one of sequences 1, 3, 9 or 21 is used.

12. Method according to claim 10,
**characterised in that**
a raw cell extract from a production organism is used.

## Revendications

1. Lyase,
**caractérisé**
**en ce qu'**elle a une séquence d'acides aminés suivant l'une des séquences 1, 3, 9 ou 21.

2. Acide désoxyribonucléique, codant pour une lyase,
**caractérisé**
**en ce qu'**il a une séquence de nucléotides suivant les séquences n° 2, 4, 10 ou 22.

3. Vecteur,
**caractérisé**
**en ce qu'**il contient une séquence suivant la revendication 2.

4. Vecteur suivant la revendication 3,
**caractérisé**
**en ce que** c'est un plasmide.

5. Vecteur suivant la revendication 4,
**caractérisé**
**en ce que** les séquences suivant la revendication 2 sont insérées dans un vecteur vide composé du groupe pET-20b(+),Pet-21a-d(+),pET-22b(+),pET-23a-d(+), pET-24a-d(+),pET-25b(+),pET-26b(+),pET-27b(+),pET-28a(+),pET-29a-c(+),pET-30a-c(+),pET-31b(+),pET-34b(+),pET-35b(+),pET-36b(+) pET-37b(+),pET-38b(+).

6. Procédé de préparation de (S)-phénylacétylcarbinol, dans lequel on fait réagir du benzaldéhyde sur du pyruvate ou sur de l'acétaldéhyde suivant la formule (1), 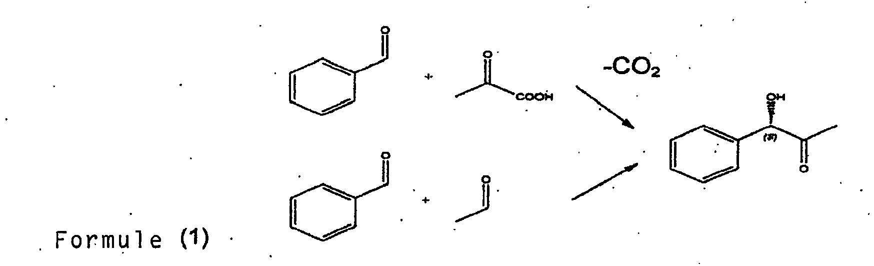 **caractérisé**
**en ce que** l'on effectue la réaction avec une lyase suivant la revendication 1.

7. Procédé suivant la revendication 6,
**caractérisé**
**en ce que** l'on effectue la production de (S)-phénylacétylcarbinol dans un organisme de production choisi dans le groupe de *e.coli, d'un corynebactérium,* par exemple de *corynebactérium glutamicum,* ou d'une levure, comme *saccharomyces cerevisiae.*

8. Procédé suivant l'une des revendications 6 ou 7,
**caractérisé**
**en ce que** l'on transforme les organismes suivant la revendication 7 par au moins un ADN des séquences 2, 4, 10 ou 22 et/ou **en ce que** l'on intègre l'ADN dans le génome.

9. Procédé suivant la revendication 8,
**caractérisé**
**en ce que**, pour la transformation, on utilise un vecteur suivant l'une des revendications 3 à 5.

10. Procédé suivant la revendication 6,
**caractérisé**
**en ce que** l'on effectue la réaction *in vitro*.

11. Procédé suivant la revendication 10,
**caractérisé**
**en ce que** l'on utilise l'enzyme isolé suivant l'une des séquences 1, 3, 9 ou 21.

12. Procédé suivant la revendication 10,
**caractérisé**
**en ce que** l'on utilise un extrait de cellule brut d'un organisme de production.


### SEQUENCE LISTING

<110> Forschungsznetrum Jülich GmbH
<120> Lyase und für die Lyase kodiediende DNA, die DNA enthaltende Vektoren, sowie Verfahren zur asymmertrischen Synthse von (S) - Phenylacetylcarbinol
<130> PT1.2666
<160> 31
<170> PatentIn version 3.5
<210> 1
   <211> 560
   <212> PRT
   <213> artificial
<220>
   <223> künstlich hergestellt
<400> 1
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln His Gly
   530 535 540
   Arg Lys Val Ala Ser Thr Asn Ala Arg Lys Thr Thr Leu Ala Leu Glu
   545 550 555 560
<210> 2
   <211> 1680
   <212> DNA
   <213> artificial
<220>
   <223> künstlich hergestellt
<400> 2
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cattggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaacatg gccgcaaggt tgcctcaacc aacgcgcgca agaccactct ggccctcgag 1680
<210> 3
   <211> 560
   <212> PRT
   <213> artificial
<220>
   <223> künstlich hergestellt
<400> 3
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ile Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Phe Gly
       530                 535                 540
   Arg Lys Val Ala Ser Thr Asn Ala Arg Lys Thr Thr Leu Ala Leu Glu
   545                 550                 555                 560
<210> 4
   <211> 1680
   <212> DNA
   <213> artificial
<220>
   <223> künstlich hergestellt
<400> 4
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg      780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaatttg gccgcaaggt tgcctcaacc aacgcgcgca agaccactct ggccctcgag    1680
<210> 5
   <211> 560
   <212> PRT
   <213> artificial
<220>
   <223> künstlich hergestellt
<400> 5
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Pro Gly
   530 535 540
   Arg Lys Val Ala Ser Thr Asn Ala Arg Lys Thr Thr Leu Ala Leu Glu
   545 550 555 560
<210> 6
   <211> 1680
   <212> DNA
   <213> artificial
<220>
   <223> künstlich hergestellt
<400> 6
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg       300
   cccaacagca atgatcaggg cacaggtcat atcctgcatc acacaatcgg caagacggat       360
   tacagctacc agcttgaaat ggcccgtcag gtcacctgtg ccgccgaaag cattaccgac       420
   gctcactccg ccccggccaa gattgaccac gtcattcgca cggcgctgcg cgagcgtaag       480
   ccggcctatc tggacatcgc gtgcaacatt gcctccgagc cctgcgtgcg gcctggccct       540
   gtcagcagcc tgctgtccga gcctgaaatc gaccacacga gcctgaaggc cgcagtggac       600
   gccacggttg ccttgctgga aaaatcggcc agccccgtca tgctgctggg cagcaagctg       660
   cgggccgcca acgcactggc cgcaaccgaa acgctggcag acaagctgca atgcgcggtg       720
   accatcatgg cggccgcgaa aggctttttc cccgaagacc acgcgggttt ccgcggcctg       780
   tactggggcg aagtctcgaa ccccggcgtg caggaactgg tggagacctc cgacgcactg       840
   ctgtgcatcg cccccgtatt caacgactat tcaacagtcg gctggtcggc atggcccaag       900
   ggccccaatg tgattctggc tgagcccgac cgcgtaacgg tcgatggccg cgcctatgac       960
   ggctttaccc tgcgcgcctt cctgcaggct ctggcggaaa aagcccccgc gcgcccggcc      1020
   tccgcacaga aaagcagcgt cccgacgtgc tcgctcaccg cgacatccga tgaagccggt      1080
   ctgacgaatg acgaaatcgt ccgtcatatc aacgccctgc tgacatcaaa cacgacgctg      1140
   gtggcagaaa ccggcgattc atggttcaat gccatgcgca tgaccctgcc gcgcggtgcg      1200
   cgcgtggaac tggaaatgca gtggggccat atcggctggt ccgtgccctc cgccttcggc      1260
   aatgccatgg gctcgcagga ccgccagcat gtggtgatgg taggcgatgg ctccttccag      1320
   cttaccgcgc aggaagtggc tcagatggtg cgctacgaac tgcccgtcat tatctttctg      1380
   atcaacaacc gtggctatgt cattggcatc gccattcatg acggcccgta caactatatc      1440
   aagaactggg attacgccgg cctgatggaa gtcttcaacg ccggagaagg ccatggactt      1500
   ggcctgaaag ccaccacccc gaaggaactg acagaagcca tcgccagggc aaaagccaat      1560
   acccgcggcc cgacgctgat cgaatgccag atcgaccgca cggactgcac ggatatgctg      1620
   gttcaacccg gccgcaaggt tgcctcaacc aacgcgcgca agaccactct ggccctcgag      1680
<210> 7
   <211> 560
   <212> PRT
   <213> artificial
<220>
   <223> künstlich hergestellt
<400> 7
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ile Gly
   530 535 540
   Arg Lys Val Ala Ser Thr Asn Ala Arg Lys Thr Thr Leu Ala Leu Glu
   545 550 555 560
<210> 8
   <211> 1680
   <212> DNA
   <213> artificial
<220>
   <223> künstlich hergestellt
<400> 8
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaaatag gccgcaaggt tgcctcaacc aacgcgcgca agaccactct ggccctcgag    1680
<210> 9
   <211> 560
   <212> PRT
   <213> artificial
<220>
   <223> künstlich hergestellt
<400> 9
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Leu Gly
   530 535 540
   Arg Lys Val Ala Ser Thr Asn Ala Arg Lys Thr Thr Leu Ala Leu Glu
   545 550 555 560
<210> 10
   <211> 1680
   <212> DNA
   <213> artificial
<220>
   <223> künstlich hergestellt
<400> 10
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cattggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaactcg gccgcaaggt tgcctcaacc aacgcgcgca agaccactct ggccctcgag 1680
<210> 11
   <211> 560
   <212> PRT
   <213> artificial
<220>
   <223> künstlich hergestellt
<400> 11
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Met Gly
   530 535 540
   Arg Lys Val Ala Ser Thr Asn Ala Arg Lys Thr Thr Leu Ala Leu Glu
   545 550 555 560
<210> 12
   <211> 1680
   <212> DNA
   <213> artificial
<220>
   <223> künstlich hergestellt
<400> 12
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc     1260
   aatgccatgg gctcgcagga ccgccagcat gtggtgatgg taggcgatgg ctccttccag     1320
   cttaccgcgc aggaagtggc tcagatggtg cgctacgaac tgcccgtcat tatctttctg     1380
   atcaacaacc gtggctatgt cattggcatc gccattcatg acggcccgta caactatatc     1440
   aagaactggg attacgccgg cctgatggaa gtcttcaacg ccggagaagg ccatggactt     1500
   ggcctgaaag ccaccacccc gaaggaactg acagaagcca tcgccagggc aaaagccaat     1560
   acccgcggcc cgacgctgat cgaatgccag atcgaccgca cggactgcac ggatatgctg     1620
   gttcaaatgg gccgcaaggt tgcctcaacc aacgcgcgca agaccactct ggccctcgag     1680
<210> 13
   <211> 560
   <212> PRT
   <213> artificial
<220>
   <223> künstlich hergestellt
<400> 13
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
             435                 440                 445
   Met Val Arg Tyr Glu Leu Pro Val Ile Ile Phe Leu Ile Asn Asn Arg
       450                 455                 460
   Gly Tyr Val Ile Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Val Gly
       530                 535                 540
   Arg Lys Val Ala Ser Thr Asn Ala Arg Lys Thr Thr Leu Ala Leu Glu
   545                 550                 555                 560
<210> 14
   <211> 1680
   <212> DNA
   <213> artificial
<220>
   <223> künstlich hergestellt
<400> 14
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag      900
   ggccccaatg tgattctggc tgagcccgac cgcgtaacgg tcgatggccg cgcctatgac      960
   ggctttaccc tgcgcgcctt cctgcaggct ctggcggaaa aagcccccgc gcgcccggcc     1020
   tccgcacaga aaagcagcgt cccgacgtgc tcgctcaccg cgacatccga tgaagccggt     1080
   ctgacgaatg acgaaatcgt ccgtcatatc aacgccctgc tgacatcaaa cacgacgctg     1140
   gtggcagaaa ccggcgattc atggttcaat gccatgcgca tgaccctgcc gcgcggtgcg     1200
   cgcgtggaac tggaaatgca gtggggccat atcggctggt ccgtgccctc cgccttcggc     1260
   aatgccatgg gctcgcagga ccgccagcat gtggtgatgg taggcgatgg ctccttccag     1320
   cttaccgcgc aggaagtggc tcagatggtg cgctacgaac tgcccgtcat tatctttctg     1380
   atcaacaacc gtggctatgt cattggcatc gccattcatg acggcccgta caactatatc     1440
   aagaactggg attacgccgg cctgatggaa gtcttcaacg ccggagaagg ccatggactt     1500
   ggcctgaaag ccaccacccc gaaggaactg acagaagcca tcgccagggc aaaagccaat     1560
   acccgcggcc cgacgctgat cgaatgccag atcgaccgca cggactgcac ggatatgctg     1620
   gttcaagtcg gccgcaaggt tgcctcaacc aacgcgcgca agaccactct ggccctcgag     1680
<210> 15
   <211> 560
   <212> PRT
   <213> artificial
<220>
   <223> künstlich hergestellt
<400> 15
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Ala Gly
   530 535 540
   Arg Lys Val Ala Ser Thr Asn Ala Arg Lys Thr Thr Leu Ala Leu Glu
   545 550 555 560
<210> 16
   <211> 1680
   <212> DNA
   <213> artificial
<220>
   <223> künstlich hergestellt
<400> 16
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaagccg gccgcaaggt tgcctcaacc aacgcgcgca agaccactct ggccctcgag    1680
<210> 17
   <211> 560
   <212> PRT
   <213> artificial
<220>
   <223> künstlich hergestellt
<400> 17
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Tyr Gly
   530 535 540
   Arg Lys Val Ala Ser Thr Asn Ala Arg Lys Thr Thr Leu Ala Leu Glu
   545 550 555 560
<210> 18
   <211> 1680
   <212> DNA
   <213> artificial
<220>
   <223> künstlich hergetellt
<400> 18
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cattggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaatacg gccgcaaggt tgcctcaacc aacgcgcgca agaccactct ggccctcgag 1680
<210> 19
   <211> 560
   <212> PRT
   <213> artificial
<220>
   <223> künstlich hergestellt
<400> 19
   Met Thr Tyr Thr Val Gly Met Tyr Leu Ala Glu Arg Leu Val Gln Ile
   1 5 10 15
   Gly Leu Lys His His Phe Ala Val Ala Gly Asp Tyr Asn Leu Val Leu
   20 25 30
   Leu Asp Gln Leu Leu Leu Asn Lys Asp Met Lys Gln Ile Tyr Cys Cys
   35 40 45
   Asn Glu Leu Asn Cys Gly Phe Ser Ala Glu Gly Tyr Ala Arg Ser Asn
   50 55 60
   Gly Ala Ala Ala Ala Val Val Thr Phe Ser Val Gly Ala Ile Ser Ala
   65 70 75 80
   Met Asn Ala Leu Gly Gly Ala Tyr Ala Glu Asn Leu Pro Val Ile Leu
   85 90 95
   Ile Ser Gly Ala Pro Asn Ser Asn Asp Gln Gly Thr Gly His Ile Leu
   100 105 110
   His His Thr Ile Gly Lys Thr Asp Tyr Ser Tyr Gln Leu Glu Met Ala
   115 120 125
   Arg Gln Val Thr Cys Ala Ala Glu Ser Ile Thr Asp Ala His Ser Ala
   130 135 140
   Pro Ala Lys Ile Asp His Val Ile Arg Thr Ala Leu Arg Glu Arg Lys
   145 150 155 160
   Pro Ala Tyr Leu Asp Ile Ala Cys Asn Ile Ala Ser Glu Pro Cys Val
   165 170 175
   Arg Pro Gly Pro Val Ser Ser Leu Leu Ser Glu Pro Glu Ile Asp His
   180 185 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ile Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Thr Gly
       530                 535                 540
   Arg Lys Val Ala Ser Thr Asn Ala Arg Lys Thr Thr Leu Ala Leu Glu
   545                 550                 555                 560
<210> 20
   <211> 1680
   <212> DNA
   <213> artificial
<220>
   <223> künstlich hergestellt
<400> 20
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaaacag gccgcaaggt tgcctcaacc aacgcgcgca agaccactct ggccctcgag    1680
<210> 21
   <211> 560
   <212> PRT
   <213> artificial
<220>
   <223> künstlich hergestellt
<400> 21
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
   485 490 495
   Gly His Gly Leu Gly Leu Lys Ala Thr Thr Pro Lys Glu Leu Thr Glu
   500 505 510
   Ala Ile Ala Arg Ala Lys Ala Asn Thr Arg Gly Pro Thr Leu Ile Glu
   515 520 525
   Cys Gln Ile Asp Arg Thr Asp Cys Thr Asp Met Leu Val Gln Gly Gly
   530 535 540
   Arg Lys Val Ala Ser Thr Asn Ala Arg Lys Thr Thr Leu Ala Leu Glu
   545 550 555 560
<210> 22
   <211> 1680
   <212> DNA
   <213> artificial
<220>
   <223> künstlich hergestellt
<400> 22
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cattggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaaggag gccgcaaggt tgcctcaacc aacgcgcgca agaccactct ggccctcgag 1680
<210> 23
   <211> 560
   <212> PRT
   <213> artificial
<220>
   <223> künstlich hergestellt
<400> 23
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
   195 200 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Ser Gly
       530                 535                 540
   Arg Lys Val Ala Ser Thr Asn Ala Arg Lys Thr Thr Leu Ala Leu Glu
   545                 550                 555                 560
<210> 24
   <211> 1680
   <212> DNA
   <213> artificial
<220>
   <223> künstlich hergestellt
<400> 24
   atgacctata ctgttggcat gtatcttgca gaacgccttg tacagatcgg gctgaagcat        60
   cacttcgccg tggcgggcga ctacaatctc gttcttctgg atcagttgct cctcaacaag       120
   gacatgaaac agatctattg ctgcaatgag ttgaactgtg gcttcagcgc ggaaggctac       180
   gcccgttcta acggggctgc ggcagcggtt gtcaccttca gcgttggcgc catttccgcc       240
   atgaacgccc tcggcggcgc ctatgccgaa aacctgccgg ttatcctgat ttccggcgcg       300
   cccaacagca atgatcaggg cacaggtcat atcctgcatc acacaatcgg caagacggat       360
   tacagctacc agcttgaaat ggcccgtcag gtcacctgtg ccgccgaaag cattaccgac       420
   gctcactccg ccccggccaa gattgaccac gtcattcgca cggcgctgcg cgagcgtaag       480
   ccggcctatc tggacatcgc gtgcaacatt gcctccgagc cctgcgtgcg gcctggccct       540
   gtcagcagcc tgctgtccga gcctgaaatc gaccacacga gcctgaaggc cgcagtggac       600
   gccacggttg ccttgctgga aaaatcggcc agccccgtca tgctgctggg cagcaagctg       660
   cgggccgcca acgcactggc cgcaaccgaa acgctggcag acaagctgca atgcgcggtg       720
   accatcatgg cggccgcgaa aggctttttc cccgaagacc acgcgggttt ccgcggcctg       780
   tactggggcg aagtctcgaa ccccggcgtg caggaactgg tggagacctc cgacgcactg       840
   ctgtgcatcg cccccgtatt caacgactat tcaacagtcg gctggtcggc atggcccaag       900
   ggccccaatg tgattctggc tgagcccgac cgcgtaacgg tcgatggccg cgcctatgac       960
   ggctttaccc tgcgcgcctt cctgcaggct ctggcggaaa aagcccccgc gcgcccggcc      1020
   tccgcacaga aaagcagcgt cccgacgtgc tcgctcaccg cgacatccga tgaagccggt      1080
   ctgacgaatg acgaaatcgt ccgtcatatc aacgccctgc tgacatcaaa cacgacgctg      1140
   gtggcagaaa ccggcgattc atggttcaat gccatgcgca tgaccctgcc gcgcggtgcg      1200
   cgcgtggaac tggaaatgca gtggggccat atcggctggt ccgtgccctc cgccttcggc      1260
   aatgccatgg gctcgcagga ccgccagcat gtggtgatgg taggcgatgg ctccttccag      1320
   cttaccgcgc aggaagtggc tcagatggtg cgctacgaac tgcccgtcat tatctttctg      1380
   atcaacaacc gtggctatgt cattggcatc gccattcatg acggcccgta caactatatc      1440
   aagaactggg attacgccgg cctgatggaa gtcttcaacg ccggagaagg ccatggactt      1500
   ggcctgaaag ccaccacccc gaaggaactg acagaagcca tcgccagggc aaaagccaat      1560
   acccgcggcc cgacgctgat cgaatgccag atcgaccgca cggactgcac ggatatgctg      1620
   gttcaaagtg gccgcaaggt tgcctcaacc aacgcgcgca agaccactct ggccctcgag      1680
<210> 25
   <211> 560
   <212> PRT
   <213> artificial
<220>
   <223> künstlich hergestellt
<400> 25
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
       210                 215                 220
   Ala Leu Ala Ala Thr Glu Thr Leu Ala Asp Lys Leu Gln Cys Ala Val
   225                 230                 235                 240
   Thr Ile Met Ala Ala Ala Lys Gly Phe Phe Pro Glu Asp His Ala Gly
                   245                 250                 255
   Phe Arg Gly Leu Tyr Trp Gly Glu Val Ser Asn Pro Gly Val Gln Glu
               260                 265                 270
   Leu Val Glu Thr Ser Asp Ala Leu Leu Cys Ile Ala Pro Val Phe Asn
           275                 280                 285
   Asp Tyr Ser Thr Val Gly Trp Ser Ala Trp Pro Lys Gly Pro Asn Val
       290                 295                 300
   Ile Leu Ala Glu Pro Asp Arg Val Thr Val Asp Gly Arg Ala Tyr Asp
   305                 310                 315                 320
   Gly Phe Thr Leu Arg Ala Phe Leu Gln Ala Leu Ala Glu Lys Ala Pro
                   325                 330                 335
   Ala Arg Pro Ala Ser Ala Gln Lys Ser Ser Val Pro Thr Cys Ser Leu
               340                 345                 350
   Thr Ala Thr Ser Asp Glu Ala Gly Leu Thr Asn Asp Glu Ile Val Arg
           355                 360                 365
   His Ile Asn Ala Leu Leu Thr Ser Asn Thr Thr Leu Val Ala Glu Thr
       370                 375                 380
   Gly Asp Ser Trp Phe Asn Ala Met Arg Met Thr Leu Pro Arg Gly Ala
   385                 390                 395                 400
   Arg Val Glu Leu Glu Met Gln Trp Gly His Ile Gly Trp Ser Val Pro
                   405                 410                 415
   Ser Ala Phe Gly Asn Ala Met Gly Ser Gln Asp Arg Gln His Val Val
               420                 425                 430
   Met Val Gly Asp Gly Ser Phe Gln Leu Thr Ala Gln Glu Val Ala Gln
           435                 440                 445
   Met Val Arg Tyr Glu Leu Pro Val Ile Ile Phe Leu Ile Asn Asn Arg
       450                 455                 460
   Gly Tyr Val Ile Gly Ile Ala Ile His Asp Gly Pro Tyr Asn Tyr Ile
   465                 470                 475                 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Cys Gly
       530                 535                 540
   Arg Lys Val Ala Ser Thr Asn Ala Arg Lys Thr Thr Leu Ala Leu Glu
   545                 550                 555                 560
<210> 26
   <211> 1680
   <212> DNA
   <213> artificial
<220>
   <223> künstlich hergestellt
<400> 26
   atgacctata ctgttggcat gtatcttgca gaacgccttg tacagatcgg gctgaagcat      60
   cacttcgccg tggcgggcga ctacaatctc gttcttctgg atcagttgct cctcaacaag     120
   gacatgaaac agatctattg ctgcaatgag ttgaactgtg gcttcagcgc ggaaggctac     180
   gcccgttcta acggggctgc ggcagcggtt gtcaccttca gcgttggcgc catttccgcc     240
   atgaacgccc tcggcggcgc ctatgccgaa aacctgccgg ttatcctgat ttccggcgcg     300
   cccaacagca atgatcaggg cacaggtcat atcctgcatc acacaatcgg caagacggat     360
   tacagctacc agcttgaaat ggcccgtcag gtcacctgtg ccgccgaaag cattaccgac     420
   gctcactccg ccccggccaa gattgaccac gtcattcgca cggcgctgcg cgagcgtaag     480
   ccggcctatc tggacatcgc gtgcaacatt gcctccgagc cctgcgtgcg gcctggccct     540
   gtcagcagcc tgctgtccga gcctgaaatc gaccacacga gcctgaaggc cgcagtggac     600
   gccacggttg ccttgctgga aaaatcggcc agccccgtca tgctgctggg cagcaagctg     660
   cgggccgcca acgcactggc cgcaaccgaa acgctggcag acaagctgca atgcgcggtg     720
   accatcatgg cggccgcgaa aggctttttc cccgaagacc acgcgggttt ccgcggcctg     780
   tactggggcg aagtctcgaa ccccggcgtg caggaactgg tggagacctc cgacgcactg     840
   ctgtgcatcg cccccgtatt caacgactat tcaacagtcg gctggtcggc atggcccaag     900
   ggccccaatg tgattctggc tgagcccgac cgcgtaacgg tcgatggccg cgcctatgac     960
   ggctttaccc tgcgcgcctt cctgcaggct ctggcggaaa aagcccccgc gcgcccggcc    1020
   tccgcacaga aaagcagcgt cccgacgtgc tcgctcaccg cgacatccga tgaagccggt    1080
   ctgacgaatg acgaaatcgt ccgtcatatc aacgccctgc tgacatcaaa cacgacgctg    1140
   gtggcagaaa ccggcgattc atggttcaat gccatgcgca tgaccctgcc gcgcggtgcg    1200
   cgcgtggaac tggaaatgca gtggggccat atcggctggt ccgtgccctc cgccttcggc    1260
   aatgccatgg gctcgcagga ccgccagcat gtggtgatgg taggcgatgg ctccttccag    1320
   cttaccgcgc aggaagtggc tcagatggtg cgctacgaac tgcccgtcat tatctttctg    1380
   atcaacaacc gtggctatgt cattggcatc gccattcatg acggcccgta caactatatc    1440
   aagaactggg attacgccgg cctgatggaa gtcttcaacg ccggagaagg ccatggactt    1500
   ggcctgaaag ccaccacccc gaaggaactg acagaagcca tcgccagggc aaaagccaat    1560
   acccgcggcc cgacgctgat cgaatgccag atcgaccgca cggactgcac ggatatgctg    1620
   gttcaatgtg gccgcaaggt tgcctcaacc aacgcgcgca agaccactct ggccctcgag    1680
<210> 27
   <211> 7041
   <212> DNA
   <213> artificial
<220>
   <223> künstlich hergestellt
<400> 27
   tggcgaatgg gacgcgccct gtagcggcgc attaagcgcg gcgggtgtgg tggttacgcg      60
   cagcgtgacc gctacacttg ccagcgccct agcgcccgct cctttcgctt tcttcccttc     120
   ctttctcgcc acgttcgccg gctttccccg tcaagctcta aatcgggggc tccctttagg     180
   gttccgattt agtgctttac ggcacctcga ccccaaaaaa cttgattagg gtgatggttc     240
   acgtagtggg ccatcgccct gatagacggt ttttcgccct ttgacgttgg agtccacgtt     300
   ctttaatagt ggactcttgt tccaaactgg aacaacactc aaccctatct cggtctattc     360
   ttttgattta taagggattt tgccgatttc ggcctattgg ttaaaaaatg agctgattta     420
   acaaaaattt aacgcgaatt ttaacaaaat attaacgttt acaatttcag gtggcacttt     480
   tcggggaaat gtgcgcggaa cccctatttg tttatttttc taaatacatt caaatatgta     540
   tccgctcatg agacaataac cctgataaat gcttcaataa tattgaaaaa ggaagagtat     600
   gagtattcaa catttccgtg tcgcccttat tccctttttt gcggcatttt gccttcctgt     660
   ttttgctcac ccagaaacgc tggtgaaagt aaaagatgct gaagatcagt tgggtgcacg     720
   agtgggttac atcgaactgg atctcaacag cggtaagatc cttgagagtt ttcgccccga     780
   agaacgtttt ccaatgatga gcacttttaa agttctgcta tgtggcgcgg tattatcccg     840
   tattgacgcc gggcaagagc aactcggtcg ccgcatacac tattctcaga atgacttggt     900
   tgagtactca ccagtcacag aaaagcatct tacggatggc atgacagtaa gagaattatg     960
   cagtgctgcc ataaccatga gtgataacac tgcggccaac ttacttctga caacgatcgg    1020
   aggaccgaag gagctaaccg cttttttgca caacatgggg gatcatgtaa ctcgccttga    1080
   tcgttgggaa ccggagctga atgaagccat accaaacgac gagcgtgaca ccacgatgcc    1140
   tgcagcaatg gcaacaacgt tgcgcaaact attaactggc gaactactta ctctagcttc    1200
   ccggcaacaa ttaatagact ggatggaggc ggataaagtt gcaggaccac ttctgcgctc    1260
   ggcccttccg gctggctggt ttattgctga taaatctgga gccggtgagc gtgggtctcg    1320
   cggtatcatt gcagcactgg ggccagatgg taagccctcc cgtatcgtag ttatctacac    1380
   gacggggagt caggcaacta tggatgaacg aaatagacag atcgctgaga taggtgcctc    1440
   actgattaag cattggtaac tgtcagacca agtttactca tatatacttt agattgattt    1500
   aaaacttcat ttttaattta aaaggatcta ggtgaagatc ctttttgata atctcatgac    1560
   caaaatccct taacgtgagt tttcgttcca ctgagcgtca gaccccgtag aaaagatcaa    1620
   aggatcttct tgagatcctt tttttctgcg cgtaatctgc tgcttgcaaa caaaaaaacc    1680
   accgctacca gcggtggttt gtttgccgga tcaagagcta ccaactcttt ttccgaaggt    1740
   aactggcttc agcagagcgc agataccaaa tactgtcctt ctagtgtagc cgtagttagg    1800
   ccaccacttc aagaactctg tagcaccgcc tacatacctc gctctgctaa tcctgttacc    1860
   agtggctgct gccagtggcg ataagtcgtg tcttaccggg ttggactcaa gacgatagtt    1920
   accggataag gcgcagcggt cgggctgaac ggggggttcg tgcacacagc ccagcttgga    1980
   gcgaacgacc tacaccgaac tgagatacct acagcgtgag ctatgagaaa gcgccacgct    2040
   tcccgaaggg agaaaggcgg acaggtatcc ggtaagcggc agggtcggaa caggagagcg    2100
   cacgagggag cttccagggg gaaacgcctg gtatctttat agtcctgtcg ggtttcgcca    2160
   cctctgactt gagcgtcgat ttttgtgatg ctcgtcaggg gggcggagcc tatggaaaaa    2220
   cgccagcaac gcggcctttt tacggttcct ggccttttgc tggccttttg ctcacatgtt    2280
   ctttcctgcg ttatcccctg attctgtgga taaccgtatt accgcctttg agtgagctga    2340
   taccgctcgc cgcagccgaa cgaccgagcg cagcgagtca gtgagcgagg aagcggaaga    2400
   gcgcctgatg cggtattttc tccttacgca tctgtgcggt atttcacacc gcatatatgg    2460
   tgcactctca gtacaatctg ctctgatgcc gcatagttaa gccagtatac actccgctat    2520
   cgctacgtga ctgggtcatg gctgcgcccc gacacccgcc aacacccgct gacgcgccct    2580
   gacgggcttg tctgctcccg gcatccgctt acagacaagc tgtgaccgtc tccgggagct    2640
   gcatgtgtca gaggttttca ccgtcatcac cgaaacgcgc gaggcagctg cggtaaagct    2700
   catcagcgtg gtcgtgaagc gattcacaga tgtctgcctg ttcatccgcg tccagctcgt    2760
   tgagtttctc cagaagcgtt aatgtctggc ttctgataaa gcgggccatg ttaagggcgg    2820
   ttttttcctg tttggtcact gatgcctccg tgtaaggggg atttctgttc atgggggtaa 2880
   tgataccgat gaaacgagag aggatgctca cgatacgggt tactgatgat gaacatgccc 2940
   ggttactgga acgttgtgag ggtaaacaac tggcggtatg gatgcggcgg gaccagagaa 3000
   aaatcactca gggtcaatgc cagcgcttcg ttaatacaga tgtaggtgtt ccacagggta 3060
   gccagcagca tcctgcgatg cagatccgga acataatggt gcagggcgct gacttccgcg 3120
   tttccagact ttacgaaaca cggaaaccga agaccattca tgttgttgct caggtcgcag 3180
   acgttttgca gcagcagtcg cttcacgttc gctcgcgtat cggtgattca ttctgctaac 3240
   cagtaaggca accccgccag cctagccggg tcctcaacga caggagcacg atcatgcgca 3300
   cccgtggggc cgccatgccg gcgataatgg cctgcttctc gccgaaacgt ttggtggcgg 3360
   gaccagtgac gaaggcttga gcgagggcgt gcaagattcc gaataccgca agcgacaggc 3420
   cgatcatcgt cgcgctccag cgaaagcggt cctcgccgaa aatgacccag agcgctgccg 3480
   gcacctgtcc tacgagttgc atgataaaga agacagtcat aagtgcggcg acgatagtca 3540
   tgccccgcgc ccaccggaag gagctgactg ggttgaaggc tctcaagggc atcggtcgag 3600
   atcccggtgc ctaatgagtg agctaactta cattaattgc gttgcgctca ctgcccgctt 3660
   tccagtcggg aaacctgtcg tgccagctgc attaatgaat cggccaacgc gcggggagag 3720
   gcggtttgcg tattgggcgc cagggtggtt tttcttttca ccagtgagac gggcaacagc 3780
   tgattgccct tcaccgcctg gccctgagag agttgcagca agcggtccac gctggtttgc 3840
   cccagcaggc gaaaatcctg tttgatggtg gttaacggcg ggatataaca tgagctgtct 3900
   tcggtatcgt cgtatcccac taccgagata tccgcaccaa cgcgcagccc ggactcggta 3960
   atggcgcgca ttgcgcccag cgccatctga tcgttggcaa ccagcatcgc agtgggaacg 4020
   atgccctcat tcagcatttg catggtttgt tgaaaaccgg acatggcact ccagtcgcct 4080
   tcccgttccg ctatcggctg aatttgattg cgagtgagat atttatgcca gccagccaga 4140
   cgcagacgcg ccgagacaga acttaatggg cccgctaaca gcgcgatttg ctggtgaccc 4200
   aatgcgacca gatgctccac gcccagtcgc gtaccgtctt catgggagaa aataatactg 4260
   ttgatgggtg tctggtcaga gacatcaaga aataacgccg gaacattagt gcaggcagct 4320
   tccacagcaa tggcatcctg gtcatccagc ggatagttaa tgatcagccc actgacgcgt 4380
   tgcgcgagaa gattgtgcac cgccgcttta caggcttcga cgccgcttcg ttctaccatc 4440
   gacaccacca cgctggcacc cagttgatcg gcgcgagatt taatcgccgc gacaatttgc 4500
   gacggcgcgt gcagggccag actggaggtg gcaacgccaa tcagcaacga ctgtttgccc 4560
   gccagttgtt gtgccacgcg gttgggaatg taattcagct ccgccatcgc cgcttccact 4620
   ttttcccgcg ttttcgcaga aacgtggctg gcctggttca ccacgcggga aacggtctga 4680
   taagagacac cggcatactc tgcgacatcg tataacgtta ctggtttcac attcaccacc 4740
   ctgaattgac tctcttccgg gcgctatcat gccataccgc gaaaggtttt gcgccattcg 4800
   atggtgtccg ggatctcgac gctctccctt atgcgactcc tgcattagga agcagcccag 4860
   tagtaggttg aggccgttga gcaccgccgc cgcaaggaat ggtgcatgca aggagatggc 4920
   gcccaacagt cccccggcca cggggcctgc caccataccc acgccgaaac aagcgctcat 4980
   gagcccgaag tggcgagccc gatcttcccc atcggtgatg tcggcgatat aggcgccagc 5040
   aaccgcacct gtggcgccgg tgatgccggc cacgatgcgt ccggcgtaga ggatcgagat 5100
   ctcgatcccg cgaaattaat acgactcact ataggggaat tgtgagcgga taacaattcc 5160
   cctctagaaa taattttgtt taactttaag aaggagatat acatatgacc tatactgttg 5220
   gcatgtatct tgcagaacgc cttgtacaga tcgggctgaa gcatcacttc gccgtggcgg 5280
   gcgactacaa tctcgttctt ctggatcagt tgctcctcaa caaggacatg aaacagatct 5340
   attgctgcaa tgagttgaac tgtggcttca gcgcggaagg ctacgcccgt tctaacgggg 5400
   ctgcggcagc ggttgtcacc ttcagcgttg gcgccatttc cgccatgaac gccctcggcg 5460
   gcgcctatgc cgaaaacctg ccggttatcc tgatttccgg cgcgcccaac agcaatgatc 5520
   agggcacagg tcatatcctg catcacacaa tcggcaagac ggattacagc taccagcttg 5580
   aaatggcccg tcaggtcacc tgtgccgccg aaagcattac cgacgctcac tccgccccgg 5640
   ccaagattga ccacgtcatt cgcacggcgc tgcgcgagcg taagccggcc tatctggaca 5700
   tcgcgtgcaa cattgcctcc gagccctgcg tgcggcctgg ccctgtcagc agcctgctgt 5760
   ccgagcctga aatcgaccac acgagcctga aggccgcagt ggacgccacg gttgccttgc 5820
   tggaaaaatc ggccagcccc gtcatgctgc tgggcagcaa gctgcgggcc gccaacgcac 5880
   tggccgcaac cgaaacgctg gcagacaagc tgcaatgcgc ggtgaccatc atggcggccg 5940
   cgaaaggctt tttccccgaa gaccacgcgg gtttccgcgg cctgtactgg ggcgaagtct 6000
   cgaaccccgg cgtgcaggaa ctggtggaga cctccgacgc actgctgtgc atcgcccccg 6060
   tattcaacga ctattcaaca gtcggctggt cggcatggcc caagggcccc aatgtgattc 6120
   tggctgagcc cgaccgcgta acggtcgatg gccgcgccta tgacggcttt accctgcgcg 6180
   ccttcctgca ggctctggcg gaaaaagccc ccgcgcgccc ggcctccgca cagaaaagca 6240
   gcgtcccgac gtgctcgctc accgcgacat ccgatgaagc cggtctgacg aatgacgaaa 6300
   tcgtccgtca tatcaacgcc ctgctgacat caaacacgac gctggtggca gaaaccggcg 6360
   attcatggtt caatgccatg cgcatgaccc tgccgcgcgg tgcgcgcgtg gaactggaaa 6420
   tgcagtgggg ccatatcggc tggtccgtgc cctccgcctt cggcaatgcc atgggctcgc 6480
   aggaccgcca gcatgtggtg atggtaggcg atggctcctt ccagcttacc gcgcaggaag 6540
   tggctcagat ggtgcgctac gaactgcccg tcattatctt tctgatcaac aaccgtggct 6600
   atgtcattgg catcgccatt catgacggcc cgtacaacta tatcaagaac tgggattacg 6660
   ccggcctgat ggaagtcttc aacgccggag aaggccatgg acttggcctg aaagccacca 6720
   ccccgaagga actgacagaa gccatcgcca gggcaaaagc caatacccgc ggcccgacgc 6780
   tgatcgaatg ccagatcgac cgcacggact gcacggatat gctggttcaa catggccgca 6840
   aggttgcctc aaccaacgcg cgcaagacca ctctggccct cgagcaccac caccaccacc 6900
   actgagatcc ggctgctaac aaagcccgaa aggaagctga gttggctgct gccaccgctg 6960
   agcaataact agcataaccc cttggggcct ctaaacgggt cttgaggggt tttttgctga 7020
   aaggaggaac tatatccgga t 7041
<210> 28
   <211> 1680
   <212> DNA
   <213> artificial
<220>
   <223> künstlich hergestellt
<400> 28
   atgacctata ctgttggcat gtatcttgca gaacgccttg tacagatcgg gctgaagcat 60
   cacttcgccg tggcgggcga ctacaatctc gttcttctgg atcagttgct cctcaacaag 120
   gacatgaaac agatctattg ctgcaatgag ttgaactgtg gcttcagcgc ggaaggctac 180
   gcccgttcta acggggctgc ggcagcggtt gtcaccttca gcgttggcgc catttccgcc 240
   atgaacgccc tcggcggcgc ctatgccgaa aacctgccgg ttatcctgat ttccggcgcg 300
   cccaacagca atgatcaggg cacaggtcat atcctgcatc acacaatcgg caagacggat 360
   tacagctacc agcttgaaat ggcccgtcag gtcacctgtg ccgccgaaag cattaccgac 420
   gctcactccg ccccggccaa gattgaccac gtcattcgca cggcgctgcg cgagcgtaag 480
   ccggcctatc tggacatcgc gtgcaacatt gcctccgagc cctgcgtgcg gcctggccct 540
   gtcagcagcc tgctgtccga gcctgaaatc gaccacacga gcctgaaggc cgcagtggac 600
   gccacggttg ccttgctgga aaaatcggcc agccccgtca tgctgctggg cagcaagctg 660
   cgggccgcca acgcactggc cgcaaccgaa acgctggcag acaagctgca atgcgcggtg 720
   accatcatgg cggccgcgaa aggctttttc cccgaagacc acgcgggttt ccgcggcctg 780
   tactggggcg aagtctcgaa ccccggcgtg caggaactgg tggagacctc cgacgcactg 840
   ctgtgcatcg cccccgtatt caacgactat tcaacagtcg gctggtcggc atggcccaag 900
   ggccccaatg tgattctggc tgagcccgac cgcgtaacgg tcgatggccg cgcctatgac 960
   ggctttaccc tgcgcgcctt cctgcaggct ctggcggaaa aagcccccgc gcgcccggcc 1020
   tccgcacaga aaagcagcgt cccgacgtgc tcgctcaccg cgacatccga tgaagccggt 1080
   ctgacgaatg acgaaatcgt ccgtcatatc aacgccctgc tgacatcaaa cacgacgctg 1140
   gtggcagaaa ccggcgattc atggttcaat gccatgcgca tgaccctgcc gcgcggtgcg 1200
   cgcgtggaac tggaaatgca gtggggccat atcggctggt ccgtgccctc cgccttcggc 1260
   aatgccatgg gctcgcagga ccgccagcat gtggtgatgg taggcgatgg ctccttccag 1320
   cttaccgcgc aggaagtggc tcagatggtg cgctacgaac tgcccgtcat tatctttctg 1380
   atcaacaacc gtggctatgt cattggcatc gccattcatg acggcccgta caactatatc 1440
   aagaactggg attacgccgg cctgatggaa gtcttcaacg ccggagaagg ccatggactt 1500
   ggcctgaaag ccaccacccc gaaggaactg acagaagcca tcgccagggc aaaagccaat 1560
   acccgcggcc cgacgctgat cgaatgccag atcgaccgca cggactgcac ggatatgctg 1620
   gttcaatggg gccgcaaggt tgcctcaacc aacgcgcgca agaccactct ggccctcgag 1680
<210> 29
   <211> 560
   <212> PRT
   <213> artificial
<220>
   <223> künstlich hergestellt
<400> 29
   Met Thr Tyr Thr Val Gly Met Tyr Leu Ala Glu Arg Leu Val Gln Ile
   1               5                   10                  15
   Gly Leu Lys His His Phe Ala Val Ala Gly Asp Tyr Asn Leu Val Leu
               20                  25                  30
   Leu Asp Gln Leu Leu Leu Asn Lys Asp Met Lys Gln Ile Tyr Cys Cys
           35                  40                  45
   Asn Glu Leu Asn Cys Gly Phe Ser Ala Glu Gly Tyr Ala Arg Ser Asn
       50                  55                  60
   Gly Ala Ala Ala Ala Val Val Thr Phe Ser Val Gly Ala Ile Ser Ala
   65                  70                  75                  80
   Met Asn Ala Leu Gly Gly Ala Tyr Ala Glu Asn Leu Pro Val Ile Leu
                   85                  90                  95
   Ile Ser Gly Ala Pro Asn Ser Asn Asp Gln Gly Thr Gly His Ile Leu
               100                 105                 110
   His His Thr Ile Gly Lys Thr Asp Tyr Ser Tyr Gln Leu Glu Met Ala
           115                 120                 125
   Arg Gln Val Thr Cys Ala Ala Glu Ser Ile Thr Asp Ala His Ser Ala
       130                 135                 140
   Pro Ala Lys Ile Asp His Val Ile Arg Thr Ala Leu Arg Glu Arg Lys
   145                 150                 155                 160
   Pro Ala Tyr Leu Asp Ile Ala Cys Asn Ile Ala Ser Glu Pro Cys Val
                   165                 170                 175
   Arg Pro Gly Pro Val Ser Ser Leu Leu Ser Glu Pro Glu Ile Asp His
               180                 185                 190
   Thr Ser Leu Lys Ala Ala Val Asp Ala Thr Val Ala Leu Leu Glu Lys
           195                 200                 205
   Ser Ala Ser Pro Val Met Leu Leu Gly Ser Lys Leu Arg Ala Ala Asn
   210 215 220
   Ala Leu Ala Ala Thr Glu Thr Leu Ala Asp Lys Leu Gln Cys Ala Val
   225 230 235 240
   Thr Ile Met Ala Ala Ala Lys Gly Phe Phe Pro Glu Asp His Ala Gly
   245 250 255
   Phe Arg Gly Leu Tyr Trp Gly Glu Val Ser Asn Pro Gly Val Gln Glu
   260 265 270
   Leu Val Glu Thr Ser Asp Ala Leu Leu Cys Ile Ala Pro Val Phe Asn
   275 280 285
   Asp Tyr Ser Thr Val Gly Trp Ser Ala Trp Pro Lys Gly Pro Asn Val
   290 295 300
   Ile Leu Ala Glu Pro Asp Arg Val Thr Val Asp Gly Arg Ala Tyr Asp
   305 310 315 320
   Gly Phe Thr Leu Arg Ala Phe Leu Gln Ala Leu Ala Glu Lys Ala Pro
   325 330 335
   Ala Arg Pro Ala Ser Ala Gln Lys Ser Ser Val Pro Thr Cys Ser Leu
   340 345 350
   Thr Ala Thr Ser Asp Glu Ala Gly Leu Thr Asn Asp Glu Ile Val Arg
   355 360 365
   His Ile Asn Ala Leu Leu Thr Ser Asn Thr Thr Leu Val Ala Glu Thr
   370 375 380
   Gly Asp Ser Trp Phe Asn Ala Met Arg Met Thr Leu Pro Arg Gly Ala
   385 390 395 400
   Arg Val Glu Leu Glu Met Gln Trp Gly His Ile Gly Trp Ser Val Pro
   405 410 415
   Ser Ala Phe Gly Asn Ala Met Gly Ser Gln Asp Arg Gln His Val Val
   420 425 430
   Met Val Gly Asp Gly Ser Phe Gln Leu Thr Ala Gln Glu Val Ala Gln
   435 440 445
   Met Val Arg Tyr Glu Leu Pro Val Ile Ile Phe Leu Ile Asn Asn Arg
   450 455 460
   Gly Tyr Val Ile Gly Ile Ala Ile His Asp Gly Pro Tyr Asn Tyr Ile
   465 470 475 480
   Lys Asn Trp Asp Tyr Ala Gly Leu Met Glu Val Phe Asn Ala Gly Glu
                   485                 490                 495
   Gly His Gly Leu Gly Leu Lys Ala Thr Thr Pro Lys Glu Leu Thr Glu
               500                 505                 510
   Ala Ile Ala Arg Ala Lys Ala Asn Thr Arg Gly Pro Thr Leu Ile Glu
           515                 520                 525
   Cys Gln Ile Asp Arg Thr Asp Cys Thr Asp Met Leu Val Gln Trp Gly
       530                 535                 540
   Arg Lys Val Ala Ser Thr Asn Ala Arg Lys Thr Thr Leu Ala Leu Glu
   545                 550                 555                 560
<210> 30
   <211> 33
   <212> DNA
   <213> artificial
<220>
   <223> Künstlich hergestellt
<220>
   <221> misc_feature
   <222> (17)..(17)
   <223> n is a, c, g, or t
<400> 30
   ggatatgctg gttcaandtg gccgcaaggt tgc 33
<210> 31
   <211> 33
   <212> DNA
   <213> artificial
<220>
   <223> künstlich hergestellt
<220>
   <221> misc_feature
   <222> (18)..(18)
   <223> n is a, c, g, or t
<400> 31
   ggcaaccttg cggccahntt gaaccagcat atc 33